# EUROPEAN PATENT APPLICATION

(11) **EP 3 354 203 A1**
(43) Date of publication of application: **01.08.2018**
(21) Application number: 16848530.8
(22) Date of filing: 12.09.2016
(51) Int. Cl.: A61B 8/12

(54) **ULTRASONIC OBSERVATION DEVICE**

(30) Priority: 25.09.2015 JP 2015188362
(71) Applicant: Olympus Corporation, Hachioji-shi Tokyo 192-8507 (JP)
(72) Inventor: MISONO, Kazuhiro, Hachioji-shi Tokyo 192-8507 (JP)
(74) Representative: Gunzelmann, Rainer
(86) International application number: PCT/JP2016/076830
(87) International publication number: WO 2017/051742

(57) **Abstract**

An ultrasound observation apparatus according to the present invention is an ultrasound observation apparatus for generating an ultrasound image based on an echo signal obtained by converting an ultrasound echo being an ultrasound wave transmitted onto an observation target and reflected by the observation target, into an electrical signal, and the ultrasound observation apparatus includes a first ultrasound processing unit configured to control transmission and reception processing of an ultrasound wave to the observation target, and generate first image data based on an echo signal obtained by the transmission and reception processing, a second ultrasound processing unit configured to control transmission and reception processing of an ultrasound wave at a timing different from that of transmission and reception processing performed by the first ultrasound processing unit, and using a scanning method having a frame rate shorter than a frame rate of the first ultrasound processing unit, and generate second image data based on an obtained echo signal, and an image synthesis processing unit configured to synthesize the first image data and the second image data.

## Description

### Field

The present invention relates to an ultrasound observation apparatus that observes observation target tissue using ultrasound waves.

### Background

For observing the property of body tissue or material that is an observation target, ultrasound waves are applied in some cases. More specifically, the observation of the observation target is performed by transmitting ultrasound waves to the observation target, generating an ultrasound image based on an ultrasound echo reflected by the observation target, and displaying the generated ultrasound image.

As a technique of performing observation and treatment of body tissue using ultrasound waves, fine-needle aspiration cytology that uses an ultrasound endoscope is known. In the fine-needle aspiration cytology, an insertion portion of the ultrasound endoscope is inserted into a subject, target tissue is punctured with a puncture needle (biopsy needle) inserted into a pipe path inside the insertion portion, while observing an ultrasound image of the inside of the subject, and the tissue is sucked and collected via the puncture needle. As a technique of displaying an ultrasound image including an ultrasound image of a puncture needle, a technique of highlighting an ultrasound image of a puncture needle is disclosed (e.g., refer to Patent Literature 1).

### Citation List

### Patent Literature

Patent Literature 1: JP 2004-208859 A

### Summary

### Technical Problem

Meanwhile, a puncture needle inside an insertion portion is curved inside a pipe path of the insertion portion to partially contact the pipe path. It is therefore difficult to transmit force to a needle tip due to static frictional force generated with the pipe path, even if an operator pushes the puncture needle. Thus, by continuously and swiftly pushing and pulling the puncture needle, and thereby causing the puncture needle to go back and forth with respect to a distal end of the insertion portion, an operator punctures target tissue with the puncture needle, to collect the tissue. At this time, if the puncture needle is caused to go back and forth at a speed higher than a frame rate of an ultrasound image, a movement of the puncture needle cannot be captured in the ultrasound image, and the puncture needle cannot be confirmed from the ultrasound image in some cases. For solving this problem, it is conceived to increase a frame rate of the ultrasound image. Nevertheless, if a frame rate is increased, resolution of the ultrasound image declines, which is not preferable from the viewpoint of observation.

The present invention has been devised in view of the foregoing, and the object of the present invention is to provide an ultrasound observation apparatus that can capture a movement of a puncture needle while maintaining resolution of an ultrasound image, even in the case of continuously and swiftly pushing and pulling the puncture needle.

### Solution to Problem

To solve the above-described problem and achieve the object, an ultrasound observation apparatus according to the present invention is an ultrasound observation apparatus for generating an ultrasound image based on an echo signal obtained by converting an ultrasound echo being an ultrasound wave transmitted onto an observation target and reflected by the observation target, into an electrical signal, and includes: a first ultrasound processing unit configured to control transmission and reception processing of an ultrasound wave to the observation target, and generate first image data based on an echo signal obtained by the transmission and reception processing; a second ultrasound processing unit configured to control transmission and reception processing of an ultrasound wave to the observation target by using a scanning method having a frame rate shorter than a frame rate of the first ultrasound processing unit at a timing different from the transmission and reception processing performed by the first ultrasound processing unit, and generate second image data based on an echo signal obtained by the transmission and reception processing; and an image synthesis processing unit configured to synthesize the first image data generated by the first ultrasound processing unit, and the second image data generated by the second ultrasound processing unit, to generate display image data.

In the above-described ultrasound observation apparatus according to the present invention, the second ultrasound processing unit generates a plurality of the second image data while the first ultrasound processing unit acquires an echo signal corresponding to one frame of the first image data.

In the above-described ultrasound observation apparatus according to the present invention, the first ultrasound processing unit outputs a transmission signal for generating a first transmission echo for performing scanning for each line extending in a predetermined direction, to an ultrasound probe that transmits and receives the ultrasound wave, and outputs the echo signal, and the second ultrasound processing unit outputs, to the ultrasound probe, a transmission signal for generating a second transmission echo having a range wider than the first transmission echo.

In the above-described ultrasound observation apparatus according to the present invention, the second transmission echo is a plane wave in which phases are two-dimensionally aligned.

In the above-described ultrasound observation apparatus according to the present invention, generation processing of the second image data that is performed by the second ultrasound processing unit includes image enhancement processing.

The above-described ultrasound observation apparatus according to the present invention further includes a detection unit configured to detect existence of a drawing target of the second image data in an image corresponding to the first image data, based on the first image data, and a control unit configured to control whether to operate the second ultrasound processing unit, according to a detection result of the detection unit.

In the above-described ultrasound observation apparatus according to the present invention, the first and second image data are each generated based on the echo signal obtained by the same ultrasound probe.

### Advantageous Effects of Invention

According to the present invention, such an effect that a movement of a puncture needle can be captured while maintaining resolution of an ultrasound image, even in the case of continuously and swiftly pushing and pulling the puncture needle is caused.

### Brief Description of Drawings

FIG. 1 is a block diagram illustrating a configuration of an ultrasound observation system including an ultrasound observation apparatus according to a first embodiment of the present invention.
FIG. 2 is a perspective view schematically illustrating an example of a distal end configuration of an insertion portion of an ultrasound endoscope according to the first embodiment of the present invention.
FIG. 3 is a diagram illustrating an ultrasound transmission timing controlled by the ultrasound observation apparatus according to the first embodiment of the present invention.
FIG. 4 is a diagram illustrating an example of a reception pattern of a signal that is based on an ultrasound echo in B mode image acquisition performed by the ultrasound observation apparatus according to the first embodiment of the present invention.
FIG. 5 is a diagram illustrating an example of a reception pattern of a signal that is based on an ultrasound echo in puncture needle image acquisition performed by the ultrasound observation apparatus according to the first embodiment of the present invention.
FIG. 6 is a perspective view schematically illustrating another example of a distal end configuration of an insertion portion of an ultrasound endoscope according to the first embodiment of the present invention.
FIG. 7 is a diagram illustrating an ultrasound wave transmitted from an ultrasound transducer in puncture needle image acquisition performed by the ultrasound observation apparatus according to the first embodiment of the present invention.
FIG. 8 is a diagram illustrating an ultrasound image obtained by control of the ultrasound observation apparatus according to the first embodiment of the present invention.
FIG. 9 is a block diagram illustrating a configuration of an ultrasound observation system including an ultrasound observation apparatus according to a second embodiment of the present invention.
FIG. 10 is a diagram illustrating an ultrasound transmission timing controlled by the ultrasound observation apparatus according to the second embodiment of the present invention.
FIG. 11 is a block diagram illustrating a configuration of an ultrasound observation system including an ultrasound observation apparatus according to a third embodiment of the present invention.
FIG. 12 is a block diagram illustrating a configuration of an ultrasound observation system including an ultrasound observation apparatus according to a fourth embodiment of the present invention.

### Description of Embodiments

A mode for carrying out the present invention (hereinafter, referred to as an "embodiment") will be described below with reference to the appended drawings. In addition, the present invention is not limited by the embodiment to be described below. Furthermore, in the description of the drawings, the same parts are assigned the same signs.

### (First Embodiment)

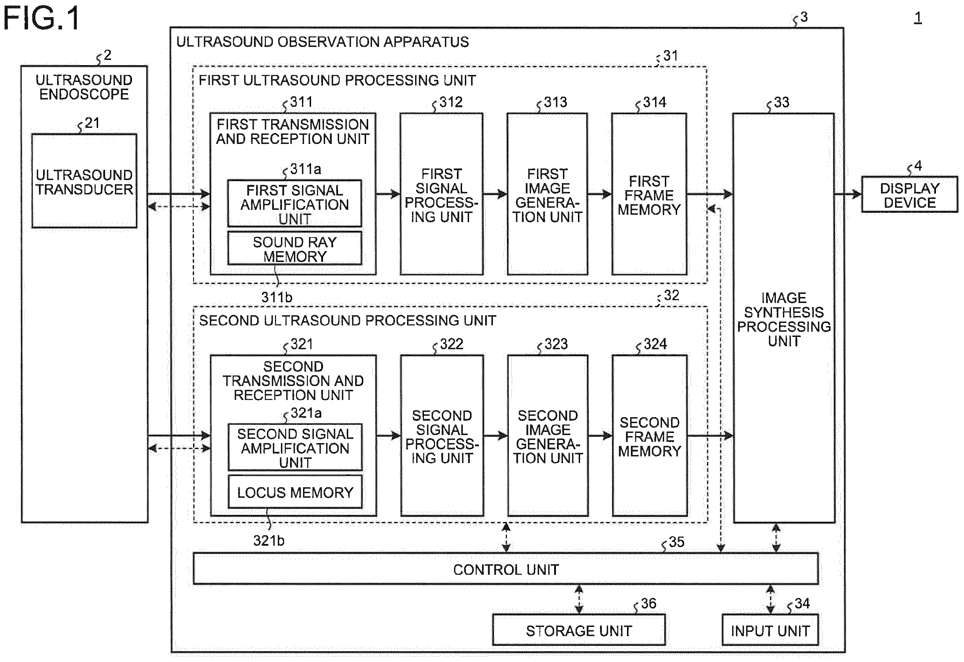

FIG. 1 is a block diagram illustrating a configuration of an ultrasound observation system including an ultrasound observation apparatus according to a first embodiment of the present invention. An ultrasound observation system 1 illustrated in FIG. 1 includes an ultrasound endoscope 2 that transmits ultrasound waves to a subject being an observation target, and receives ultrasound waves reflected by the subject, an ultrasound observation apparatus 3 that generates an ultrasound image based on an ultrasound signal acquired by the ultrasound endoscope 2, and a display device 4 that displays the ultrasound image generated by the ultrasound observation apparatus 3. In the block diagram illustrated in FIG. 1, solid-line arrows indicate transmission of signals related to image data, and broken-line arrows indicate transmission of signals related to control.

The ultrasound endoscope 2 includes an insertion portion having flexibility, and includes, at a distal end portion of the insertion portion, an ultrasound transducer 21 that converts an electrical pulse signal received from the ultrasound observation apparatus 3, into an ultrasound pulse (acoustic pulse), emits the ultrasound pulse onto a subject, converts an ultrasound echo reflected by the subject, into an electrical echo signal represented by voltage change, and outputs the electrical echo signal. The ultrasound transducer 21 may be any of a convex transducer, a linear transducer, and a radial transducer. The ultrasound endoscope 2 may mechanically perform scanning of the ultrasound transducer 21, or may electrically perform scanning by providing, as the ultrasound transducer 21, a plurality of elements in an array, and electrically switching an element involved in transmission and reception, or delaying transmission and reception of each element.

The ultrasound endoscope 2 normally includes an imaging optical system and an image sensor, is inserted into a digestive tract (esophagus, stomach, duodenum, large intestine) or a respiratory organ (trachea, bronchus) of the subject, and can perform capturing of the digestive tract, the respiratory organ, or their adjacent organs (pancreas, cholecystis, biliary duct, biliary tract, lymph nodes, mediastinum organ, blood vessels, etc.). In addition, their adjacent organs (pancreas, cholecystis, biliary duct, biliary tract, lymph nodes, mediastinum organ, blood vessels, etc.) can be captured using ultrasound waves. In addition, the ultrasound endoscope 2 includes a light guide that guides illumination light to be emitted onto a subject when optical capturing is performed. The light guide has a distal end portion and a proximal end portion. The distal end portion reaches a distal end of an insertion portion to be inserted into a subject of the ultrasound endoscope 2. On the other hand, the proximal end portion is connected to a light source device that generates illumination light.

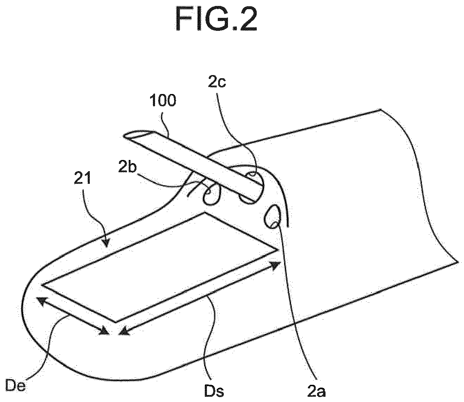

FIG. 2 is a perspective view schematically illustrating an example of a distal end configuration of an insertion portion of an ultrasound endoscope according to this first embodiment. As illustrated in FIG. 2, a distal end portion of the ultrasound endoscope 2 includes an illumination lens 2a that collects illumination light and emits the illumination light to the outside, an objective lens 2b that constitutes a part of the imaging optical system, and takes in light from the outside, and a processing tool protrusion port 2c that is communicated with a processing tool insertion path formed in the ultrasound endoscope 2, and causes a processing tool to protrude from the distal end of the insertion portion. In this first embodiment, the description will be given as an example while assuming that the ultrasound transducer 21 is a linear-type ultrasound transducer as illustrated in FIG. 2, and is a one-dimensional array (1D array) in which a plurality of piezoelectric elements is arrayed in a line. Hereinafter, as illustrated in FIG. 2, a longitudinal direction of the piezoelectric elements will be referred to as an elevation direction De, and an array direction of the piezoelectric elements will be referred to as a scanning direction Ds.

The ultrasound observation apparatus 3 includes a first ultrasound processing unit 31 that performs ultrasound transmission and reception processing for generating a B mode image, a second ultrasound processing unit 32 that performs ultrasound transmission and reception processing for generating an ultrasound image of a puncture needle (hereinafter, referred to as a puncture needle image), an image synthesis processing unit 33 that synthesizes the B mode image generated by the first ultrasound processing unit 31, and the puncture needle image generated by the second ultrasound processing unit 32, an input unit 34 that is implemented by using a user interface such as a keyboard, a mouse, and a touch panel, and receives input of various types of information, a control unit 35 that controls the entire ultrasound observation system 1, and a storage unit 36 that stores various types of information necessary for an operation of an ultrasound diagnosis apparatus 3.

The first ultrasound processing unit 31 includes a first transmission and reception unit 311 that is electrically connected with the ultrasound endoscope 2, transmits, to the ultrasound transducer 21, a transmission signal (pulse signal) including a high-voltage pulse, based on a predetermined waveform and transmission timing, and receives an echo signal being an electrical reception signal, from the ultrasound transducer 21, to generate and output data of a digital high-frequency (RF: Radio Frequency) signal (hereinafter, referred to as RF data), a first signal processing unit 312 that generates digital B mode reception data based on the RF data received from the first transmission and reception unit 311, a first image generation unit 313 that generates B mode image data based on the B mode reception data generated by the first signal processing unit 312, and a first frame memory 314 that stores the B mode image data generated by the first image generation unit 313.

The first transmission and reception unit 311 is electrically connected with the ultrasound endoscope 2, transmits, to the ultrasound transducer 21, a transmission signal (pulse signal) including a high-voltage pulse, based on a predetermined waveform and transmission timing, and receives an echo signal being an electrical reception signal, from the ultrasound transducer 21.

The first transmission and reception unit 311 includes a first signal amplification unit 311a that amplifies an echo signal, and a sound ray memory 311b that stores the echo signal amplified by the first signal amplification unit 311a. The first signal amplification unit 311a receives the echo signal from the first transmission and reception unit 311, to generate and output RF data. The first signal amplification unit 311a performs Sensitivity Time Control (STC) correction that amplifies an echo signal with a larger reception depth using a higher amplification rate, applies processing such as filtering to the amplified echo signal, and then performs A/D conversion. The first signal amplification unit 311a thereby generates RF data in a time domain, and outputs the RF data to the sound ray memory 311b. The sound ray memory 311b stores sound ray data corresponding to a signal being an echo signal amplified by the first signal amplification unit 311a that has been received by each piezoelectric element.

The first signal processing unit 312 generates digital B mode reception data for each RF data received from the first transmission and reception unit 311, more specifically, for each sound ray data corresponding to one sound ray that has been read from the sound ray memory 311b. The first signal processing unit 312 applies known processing such as bandpass filter, envelope detection, and logarithmic conversion, on the RF data, to generate digital B mode reception data. In the logarithmic conversion, a common logarithm of an amount obtained by dividing the RF data by reference voltage V_{c} is obtained, and represented by a decibel value. In the B mode reception data, amplitudes or intensities of reception signals that indicate the intensity of reflection of ultrasound pulses are arranged along a transmission and reception direction (depth direction) of ultrasound pulses. The first signal processing unit 312 outputs the generated B mode reception data to the first image generation unit 313. The first signal processing unit 312 is implemented by using a central processing unit (CPU), various arithmetic circuits, or the like.

The first image generation unit 313 generates frame data by converting a scan direction of the B mode reception data received from the first signal processing unit 312, and generates B mode image data based on the frame data.

More specifically, the first image generation unit 313 converts the scan direction of the B mode reception data from a scan direction of ultrasound waves to a display direction of the display device 4. After that, the first image generation unit 313 generates B mode image data including a B mode image being an ultrasound image that displays the amplitude of an echo signal with being converted into brightness. The first image generation unit 313 performs image processing that uses a known technique such as gain processing and contrast processing, on the frame data, and generates B mode image data by performing data thinning or the like that corresponds to a data step width defined according to a display range of an image in the display device 4. The B mode image is a gray scale image having matched values of red (R), green (G), and blues (B), which are variables used in a case in which an RGB color system is employed as a color space.

The first image generation unit 313 applies coordinate conversion of performing rearrangement so that a scan range can be represented spatially-correctly, on the B mode reception data from the first signal processing unit 312, and then, applies interpolation processing between B mode reception data. The first image generation unit 313 thereby fills spaces between the B mode reception data, to generate B mode image data.

The first frame memory 314 is implemented by using a ring buffer, for example, and stores B mode image data with a certain amount (predetermined number of frames) that have been generated by the first image generation unit 313, in chronological order. If capacity becomes insufficient, (if B mode image data with a predetermined number of frames are stored), latest B mode images with a predetermined number of frames are stored in chronological order by overwriting the oldest B mode image data with the latest B mode image data.

The second ultrasound processing unit 32 includes a second transmission and reception unit 321 that is electrically connected with the ultrasound endoscope 2, transmits, to the ultrasound transducer 21, a transmission signal (pulse signal) including a high-voltage pulse, based on a predetermined waveform and transmission timing, and receives an echo signal being an electrical reception signal, from the ultrasound transducer 21, to generate and output RF data, a second signal processing unit 322 that generates digital puncture needle reception data based on the RF data received from the second transmission and reception unit 321, a second image generation unit 323 that generates puncture needle image data based on the puncture needle reception data generated by the second signal processing unit 322, and a second frame memory 324 that stores the puncture needle image data generated by the second image generation unit 323.

The second transmission and reception unit 321 is electrically connected with the ultrasound endoscope 2, transmits, to the ultrasound transducer 21, a transmission signal (pulse signal) including a high-voltage pulse, based on a predetermined waveform and transmission timing, and receives an echo signal being an electrical reception signal, from the ultrasound transducer 21.

The second transmission and reception unit 321 includes a second signal amplification unit 321a that amplifies an echo signal, and a locus memory 321b that stores the echo signal amplified by the second signal amplification unit 321a. The second signal amplification unit 321a receives the echo signal from the second transmission and reception unit 321, to generate and output RF data. The second signal amplification unit 321a performs Sensitivity Time Control (STC) correction that amplifies an echo signal with a larger reception depth using a higher amplification rate, applies processing such as filtering on the amplified echo signal, and then performs A/D conversion. The second signal amplification unit 321a thereby generates RF data in a time domain, and outputs the RF data to the locus memory 321b. The locus memory 321b stores sound ray data corresponding to a signal being an echo signal amplified by the second signal amplification unit 321a that has been received by each piezoelectric element.

The second signal processing unit 322 generates digital puncture needle reception data for each RF data received from the second transmission and reception unit 321, more specifically, for each sound ray data corresponding to one frame that has been read from the locus memory 321b. The second signal processing unit 322 applies known processing such as bandpass filter, envelope detection, and logarithmic conversion, on the RF data, to generate digital puncture needle reception data. In the logarithmic conversion, a common logarithm of an amount obtained by dividing the RF data by reference voltage V_{c} is obtained, and represented by a decibel value. The second signal processing unit 322 outputs the generated puncture needle reception data to the second image generation unit 323. The second signal processing unit 322 is implemented by using a central processing unit (CPU), various arithmetic circuits, or the like.

The second image generation unit 323 generates frame data by converting a scan direction of the puncture needle reception data received from the second signal processing unit 322, and generates puncture needle image data based on the frame data.

More specifically, the second image generation unit 323 converts the scan direction of the puncture needle reception from a scan direction of ultrasound waves to a display direction of the display device 4. After that, a second image generation unit 313 generates puncture needle image data including a puncture needle image being an ultrasound image that displays the amplitude of an echo signal with being converted into brightness. The second image generation unit 323 performs image processing that uses a known technique such as gain processing and contrast processing, on the frame data, and generates puncture needle image data by performing data thinning or the like that corresponds to a data step width defined according to a display range of an image in the display device 4. The puncture needle image data is a gray scale image having matched values of red (R), green (G), and blues (B), which are variables used in a case in which an RGB color system is employed as a color space.

The second image generation unit 323 applies coordinate conversion of performing rearrangement so that a scan range can be represented spatially-correctly, on the puncture needle reception data from the second signal processing unit 322, and then, applies interpolation processing between puncture needle reception data. The second image generation unit 323 thereby fills spaces between the puncture needle reception data, to generate puncture needle image data.

The second frame memory 324 is implemented by using a ring buffer, for example, and stores puncture needle image data with a certain amount (predetermined number of frames) that have been generated by the second image generation unit 323, in chronological order. If capacity becomes insufficient, (if puncture needle image data with a predetermined number of frames are stored), latest puncture needle images with a predetermined number of frames are stored in chronological order by overwriting the oldest puncture needle image data with the latest puncture needle image data.

The image synthesis processing unit 33 refers to the first frame memory 314 and the second frame memory 324, and synthesizes the B mode image data generated by the first image generation unit 313, and the puncture needle image data generated by the second image generation unit 323, to generate synthesized image data.

The input unit 34 receives input of various types of information that have been input via a user interface such as a keyboard, a mouse, and a touch panel.

The control unit 35 controls the entire ultrasound observation system 1. The control unit 35 is implemented by using a CPU, various arithmetic circuits, and the like that have calculation and control functions. The control unit 35 reads information stored by the storage unit 36, from the storage unit 36, and comprehensively controls the ultrasound observation apparatus 3 by executing various types of calculation processing related to an actuation method of the ultrasound observation apparatus 3. In addition, the control unit 35 can also be formed by using a CPU and the like that are common to the first signal processing unit 312 and/or the second signal processing unit 322.

The storage unit 36 stores various types of information necessary for an operation of the ultrasound observation apparatus 3. The storage unit 36 stores information related to a transmission timing of ultrasound waves for acquisition of a B mode image, and information related to a transmission pattern of ultrasound waves (formation pattern of transmission waves) for acquisition of a puncture needle image. Aside from the above, the storage unit 36 stores information or the like that is necessary for amplification processing, for example.

In addition, the storage unit 36 stores various programs including an actuation program for executing an actuation method of the ultrasound observation apparatus 3. The actuation program can also be widely-distributed by being recorded on a computer-readable recording medium such as a hard disc, a flash memory, a CD-ROM, a DVD-ROM, and a flexible disk. In addition, the aforementioned various programs can also be acquired by being downloaded via a communication network. The communication network here is implemented by, for example, an existing public line network, a local area network (LAN), a wide area network (WAN), or the like, and may be a wired network or a wireless network.

The storage unit 36 having the above configuration is implemented by using a read only memory (ROM) on which various programs and the like are preinstalled, a random access memory (RAM) storing calculation parameters, data, and the like of each processing, and the like.

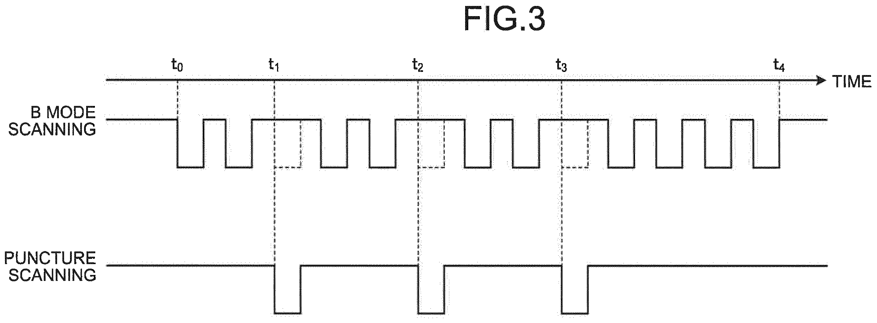

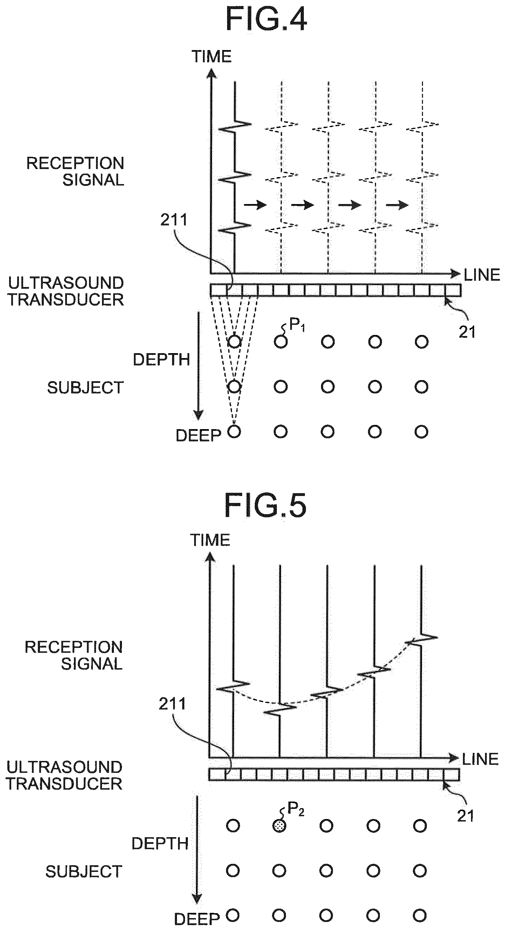

Subsequently, generation processing of B mode image data and puncture needle image data that is performed by the ultrasound observation apparatus 3 according to this first embodiment will be described with reference to FIGS. 3 to 8. FIG. 3 is a diagram illustrating an ultrasound transmission timing controlled by the ultrasound observation apparatus according to this first embodiment. FIG. 4 is a diagram illustrating an example of a reception pattern of a signal that is based on an ultrasound echo in B mode image acquisition performed by the ultrasound observation apparatus according to this first embodiment. In addition, in this first embodiment, the description will be given assuming that image data are generated based on echo signals obtained by the ultrasound transducer 21 of the same ultrasound endoscope 2. Nevertheless, image data may be created based on echo signals obtained using different ultrasound endoscopes 2.

In this first embodiment, when an ultrasound image (synthesized image) including a puncture needle 100 is generated, after B mode image data and puncture needle image data are each generated, the generated B mode image data and the puncture needle image data are synthesized, and synthesized image data is generated. At this time, a transmission timing of an ultrasound wave for generating the B mode image data, and a transmission timing of an ultrasound wave for generating the puncture needle image data are different.

The first ultrasound processing unit 31 acquires a reception signal corresponding to each line (amplitude or intensity in each position in the depth direction) at a transmission timing of B mode scanning as illustrated in FIG. 3, and generates B mode image data based on the acquired reception signal. More specifically, first, the first transmission and reception unit 311 transmits, to the ultrasound transducer 21, a transmission signal (pulse signal) including a high-voltage pulse, for each line, based on a transmission timing corresponding to the transmission timing of the B mode scanning. For example, as illustrated in FIG. 3, the ultrasound transducer 21 transmits an ultrasound wave from a piezoelectric element 211 for generating the first sound ray at a time t₀, and by sequentially transmitting ultrasound waves from the respective piezoelectric elements 211 along the scanning direction Ds, ultrasound transmission processing corresponding to one frame is completed at a time t₄. In this manner, by each of the piezoelectric elements 211 transmitting an ultrasound wave, a reception signal having a peak at each depth where a reflector exists, such as a position P₁ in a certain depth as illustrated in FIG. 4 can be obtained for each line. The depth here refers to a distance (depth) from a surface of the ultrasound transducer 21 in a normal direction of the surface. The signal received by the ultrasound transducer 21 through such ultrasound transmission processing is stored in the sound ray memory 311b of the first transmission and reception unit 311. The first signal processing unit 312 performs filter processing and detection processing for each echo signal stored in the sound ray memory 311b, to generate B mode reception data. The transmission timing of the B mode scanning is stored in the storage unit 36 as sound ray information. In addition, at the transmission timing illustrated in FIG. 3, the first ultrasound processing unit 31 performs scanning corresponding to one line at each of a fall and a rise of a pulse. FIG. 3 illustrates such a pulse signal that operations corresponding to 20 lines are performed. Actually, line scanning corresponding to 100 to 1000 times is performed. In addition, one line scanning in the B mode scanning requires about several hundreds of microseconds, and it takes about several tens to several hundreds of milliseconds for performing line scanning corresponding to one frame.

If B mode reception data is input from the first signal processing unit 312, the first image generation unit 313 generates B mode image data. The generated B mode image data is stored in the first frame memory 314.

In contrast to this, as illustrated in FIG. 3, the second ultrasound processing unit 32 acquires a reception signal corresponding to a predetermined plane wave, at a transmission timing of puncture scanning that is synchronized with the transmission timing of B mode scanning, and at a frame rate shorter than a frame rate of B mode scanning, and generates puncture needle image data based on the acquired reception signal. More specifically, first, the second transmission and reception unit 321 transmits a transmission signal (pulse signal) to the ultrasound transducer 21 based on a transmission timing corresponding to the transmission timing of puncture scanning. For example, as illustrated in FIG. 3, in the ultrasound transducer 21, by transmitting ultrasound waves as plane waves using a plurality of piezoelectric elements, at a time t₁, ultrasound transmission processing corresponding to one frame of puncture needle image data is completed. By intermittently performing similar ultrasound transmission processing at times t₂ and t₃, during ultrasound transmission processing for generating one frame of B mode image data, a reception signal for generating puncture needle image data corresponding to a plurality of frames is acquired. After the ultrasound transducer 21 receives an echo signal reflected through such ultrasound transmission processing, puncture needle image data is generated through the second signal processing unit 322 and the second image generation unit 323.

FIG. 5 is a diagram illustrating an example of a reception pattern of a signal that is based on an ultrasound echo in puncture needle image acquisition performed by the ultrasound observation apparatus according to the first embodiment of the present invention. FIG. 5 illustrates a reception echo (locus memory) pattern of each of the piezoelectric elements 211 at a position P₂ in a predetermined depth that is obtained as a reception signal in one frame scanning using plane waves. The ultrasound transducer 21 simultaneously drives piezoelectric elements, and transmits ultrasound waves so as to become plane waves perpendicular to a normal direction of the surface of the ultrasound transducer 21, and plane waves progressing in a state in which phases are two-dimensionally aligned. The ultrasound transducer 21 thereby transmits plane waves perpendicular to the normal line, to a subject, and receives ultrasound waves reflected by a reflector of the subject. In this manner, the second transmission and reception unit 321 causes the ultrasound transducer 21 to transmit a transmission echo having a two-dimensionally-wider range than a transmission echo transmitted by the first transmission and reception unit 311.

In the locus memory 321b, for each position in a predetermined depth, information of an echo signal that is extracted based on a delay amount (delay time) corresponding to a distance from the position to each piezoelectric element is stored as position data. More specifically, when a reception intensity value of the position P₂ that is based on the received echo signal is generated, as illustrated in FIG. 5, among echo signals received by the respective piezoelectric elements, a difference (delay) in reception timing occurs (a piezoelectric element having a short distance from the position P₂ first receives an echo signal). Thus, if the second signal processing unit 322 reads position data of the position P₂ from the locus memory 321b, and synthesizes reception signals received by the respective piezoelectric elements, considering a delay amount based on the position data, a reception intensity value that is based on an ultrasound wave reflected at the position P₂ can be generated. A reception intensity value at each position can be generated in this manner. Thus, if the second signal processing unit 322 generates puncture needle reception data based on reception intensity values of a plurality of positions that corresponds to a position where the puncture needle 100 performs a back-and-forth operation, and the second image generation unit 323 generates puncture needle image data based on the puncture needle reception data, images in a plane (line) parallel to a back-and-forth direction of the puncture needle 100 are synthesized, and a puncture needle image obtained by plane waves can be acquired.

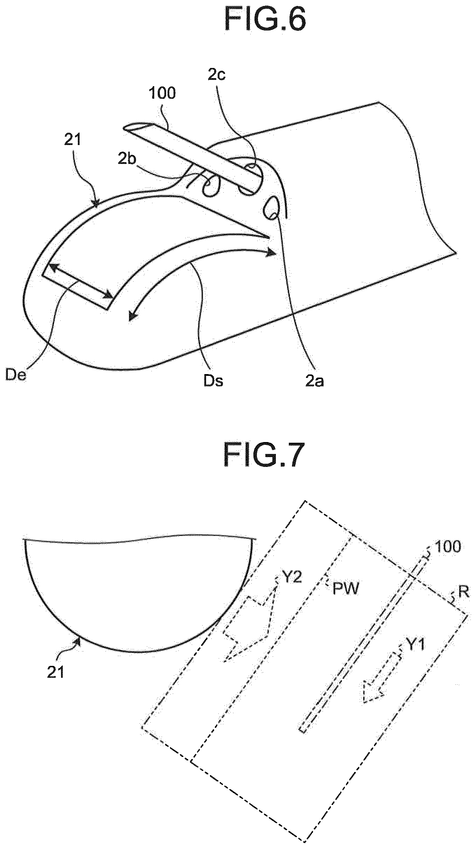

FIG. 6 is a perspective view schematically illustrating another example of a distal end configuration of an insertion portion of an ultrasound endoscope according to this first embodiment. In the aforementioned FIG. 2, the linear-type ultrasound transducer 21 has been described. Nevertheless, the ultrasound transducer 21 may be a convex-type ultrasound transducer as illustrated in FIG. 6. In the convex-type ultrasound transducer 21, by transmitting ultrasound waves while making a transmission timing of a plane wave perpendicular to the normal direction of the surface of the ultrasound transducer 21 that is a transmission timing of a piezoelectric element positioned on the normal direction, the slowest, an ultrasound wave can be transmitted as a plane wave perpendicular to the normal line.

FIG. 7 is a diagram illustrating an ultrasound wave transmitted from an ultrasound transducer in puncture needle image acquisition performed by the ultrasound observation apparatus according to the first embodiment of the present invention. By transmitting ultrasound waves while varying transmission timings of the respective piezoelectric elements as mentioned above, the ultrasound transducer 21 transmits a plane wave PW as illustrated in FIG. 7. The plane wave PW is parallel to a back-and-forth direction Y1 of the puncture needle 100, and progresses from the ultrasound transducer 21 in a direction (arrow Y2) perpendicular to the plane wave PW, in a state in which phases are two-dimensionally aligned. In addition, although the plane wave PW is preferably parallel to the back-and-forth direction Y1 of the puncture needle 100, the plane wave PW needs not be parallel thereto.

In the puncture scanning according to this first embodiment, at the time of a fall or a rise of a pulse, the ultrasound transducer 21 transmits the aforementioned plane wave PW, and receives an ultrasound echo reflected by the puncture needle 100. In other words, in the puncture scanning, during transmission processing of acquiring an echo signal corresponding to one line by the B mode scanning, an echo signal corresponding to one frame of puncture needle image data can be acquired. At this time, the puncture needle image data is only required to include at least a back-and-forth region of the puncture needle 100, and as illustrated in FIG. 7, for example, such a plane wave PW that an ultrasound echo having a scanning region R determined by a display range of the display device 4, and an entry angle of the puncture needle 100 is received is transmitted. In addition, a transmitted wave is not limited to a plane wave as long as an image of the puncture needle 100 can be drawn, and may be a spherical wave.

By performing the puncture scanning according to this first embodiment, in the B mode scanning, a time taken for line scanning increases by the number of times of puncture scanning, but a time corresponding to the increase is about several tens to several hundreds of microseconds, and can be regarded as a substantially-equivalent frame rate as compared with a case of obtaining only B mode image data.

If RF data that is based on an ultrasound echo obtained by the plane wave PW is input, the second signal processing unit 322 generates and binarizes puncture needle reception data, and then, outputs the puncture needle reception data to the second image generation unit 323. The second image generation unit 323 performs detection of the puncture needle 100 from the binarized puncture needle reception data, and generates puncture needle image data in which the puncture needle 100 is enhanced, or puncture needle image data in which only an image of the puncture needle 100 is drawn. The generated puncture needle image data is stored in the second frame memory 324.

In addition, generally, in scanning that uses the plane wave PW or aperture synthesis processing, the intensity of ultrasound waves is small, and correlation processing needs to be performed between frames. Nevertheless, in the case of detecting and drawing the puncture needle 100 as in this first embodiment, an image of the puncture needle 100 is drawn with high brightness. Thus, there is no need to perform correlation processing between frames, and puncture needle image data can be generated by scanning corresponding to one frame (one transmission processing of the plane wave PW).

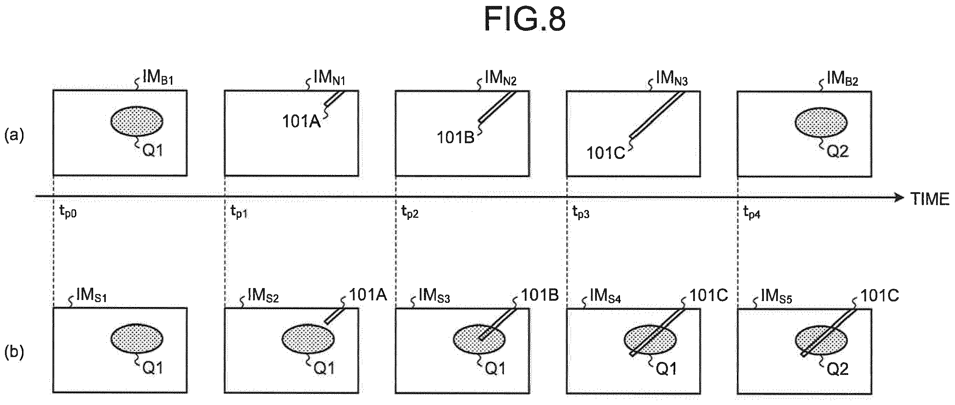

FIG. 8 is a diagram illustrating an ultrasound image obtained by control of the ultrasound observation apparatus according to this first embodiment, (a) of FIG. 8 illustrates image data generated by the first ultrasound processing unit 31 and the second ultrasound processing unit 32, and (b) of FIG. 8 illustrates synthesized image data generated by the image synthesis processing unit 33.

As illustrated in (a) of FIG. 8, if image data generated by the first ultrasound processing unit 31 and the second ultrasound processing unit 32 are arranged in the order of start of ultrasound transmission processing, B mode image data IM_{B1} in a certain frame (e.g. a time t_{P0} in FIG. 3), next, puncture needle image data IM_{N1} (e.g., a time t_{P1} in FIG. 3) generated according to the plane wave PW transmitted while line data of the B mode image data IM_{B1} is being acquired, puncture needle image data IM_{N2} (e.g., a time t_{P2} in FIG. 3) and puncture needle image data IM_{N3} (e.g., a time t_{P3} in FIG. 3), and next, B mode image data IM_{B2} related to the next frame of the B mode image data IM_{B1} are obtained. In the B mode image data IM_{B1} and the B mode image data IM_{B2}, puncture target tissues Q1 and Q2 are drawn (the tissues Q1 and Q2 are the same tissue), and in the puncture needle image data IM_{N1} to the puncture needle image data IM_{N3}, puncture needle images 101A to 101C being ultrasound images of the puncture needle 100 are drawn.

The image synthesis processing unit 33 synthesizes the B mode image data IM_{B1} and the puncture needle image data IM_{N1} to generate synthesized image data IM_{S2} (refer to (b) of FIG. 8). The image synthesis processing unit 33 similarly synthesizes the B mode image data IM_{B1} and the puncture needle image data IM_{N2} to generate synthesized image data IM_{S3}. The image synthesis processing unit 33 similarly synthesizes the B mode image data IM_{B1} and the puncture needle image data IM_{N3} to generate synthesized image data IM_{S4}. In this manner, ultrasound image data including ultrasound images of the puncture needle 100 at the times t_{P1} to t_{P3} can be acquired. In addition, because no puncture image data exists at the time t_{P0}, image data only including B mode image data is output as synthesized image data (synthesized image data IM_{S1}). In addition, also at a time t_{P4}, puncture image data corresponding to the time does not exist. Thus, synthesized image data obtained by synthesizing the B mode image data IM_{B2} and the puncture needle image data IM_{N3} being the latest puncture needle image data at the time t_{P4} is output as synthesized image data (synthesized image data IM_{S5}) at the time t_{P4}.

In this manner, the image synthesis processing unit 33 generates synthesized image data including ultrasound images of the puncture needle 100 that have a frame rate shorter than a frame rate of B mode image data. Between frame rates of consecutive B mode image data, a plurality of ultrasound image data that captures the puncture needle 100 going back and forth with respect to tissue can be thereby generated.

Synthesized image generated by the aforementioned image synthesis processing is stored in the storage unit 36 and displayed by the display device 4, under the control of the control unit 35. In addition, if the ultrasound observation apparatus 3 receives a new echo signal from the ultrasound endoscope 2, image data of the latest frame of the first frame memory 314 and/or the second frame memory 324 is updated, and image synthesis processing using the updated latest image data is performed.

According to this first embodiment described above, during line scanning for generating B mode image data of one frame that is performed by the first ultrasound processing unit 31, the second ultrasound processing unit 32 transmits the plane wave PW, and generates a plurality of puncture needle image data in which the puncture needle 100 is drawn, and the image synthesis processing unit 33 synthesizes B mode image data and puncture needle image data. Synthesized image data including ultrasound images of the puncture needle 100 that have a frame rate shorter than a frame rate of B mode image data is thereby generated. Between frame rates of consecutive B mode image data, a plurality of ultrasound image data that captures the puncture needle 100 going back and forth with respect to tissue are thereby generated. Even in the case of continuously and swiftly pushing and pulling the puncture needle 100, a movement of the puncture needle 100 can be captured while maintaining resolution of an ultrasound image.

In addition, according to this first embodiment, the second ultrasound processing unit 32 transmits the plane wave PW, and acquires a reception signal for puncture needle image data in which the puncture needle 100 is drawn. Thus, as compared with a case of acquiring a signal for each line as in B mode scanning, phase alignment can be easily performed.

### (Second Embodiment)

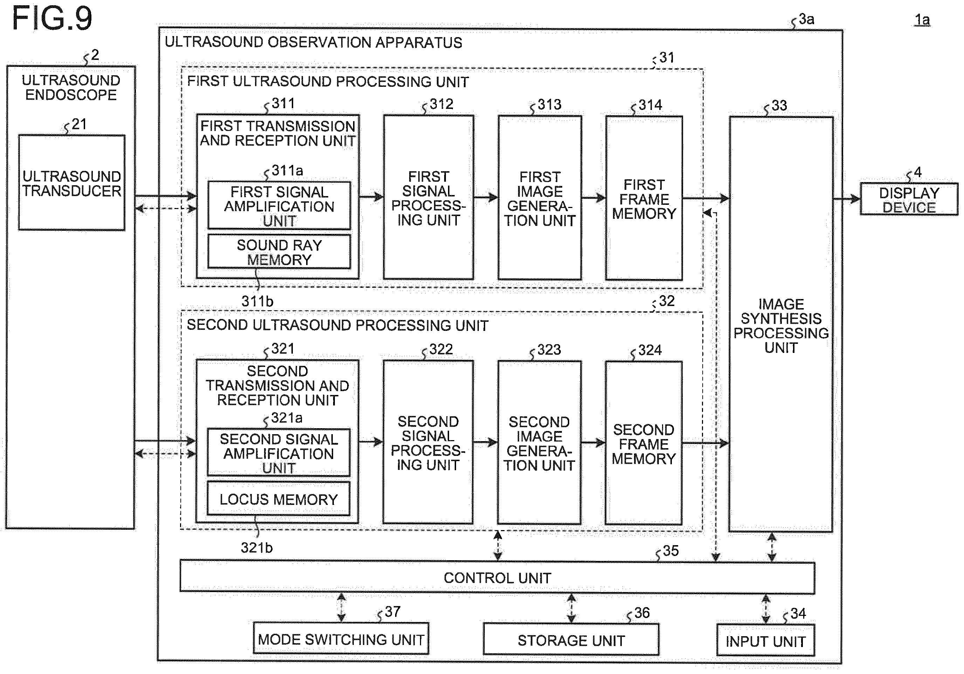

FIG. 9 is a block diagram illustrating a configuration of an ultrasound observation system including an ultrasound observation apparatus according to a second embodiment of the present invention. In the aforementioned first embodiment, the description has been given assuming that the first ultrasound processing unit 31 performs processing for generating B mode image data. In this second embodiment, the first ultrasound processing unit 31 generates image data of a flow observation mode. In the flow observation mode, flow image data in which identification information related to the existence or non-existence of a blood flow, a blood flow amount, a blood flow direction, and the like is superimposed on a B mode image is displayed on the display device 4.

An ultrasound observation system 1a according to this second embodiment includes an ultrasound endoscope 2 that transmits ultrasound waves to a subject being an observation target, and receives ultrasound waves reflected by the subject, an ultrasound observation apparatus 3a that generates an ultrasound image based on an ultrasound signal acquired by the ultrasound endoscope 2, and the display device 4 that displays the ultrasound image generated by the ultrasound observation apparatus 3a. The ultrasound observation apparatus 3a includes a mode switching unit 37 in addition to the aforementioned configurations of the ultrasound observation apparatus 3.

If the input unit 34 receives mode switch instruction information indicating that an observation mode is to be switched, the mode switching unit 37 switches an observation mode to a B mode image observation mode or a flow observation mode based on the mode switch instruction information.

If the observation mode is set to the B mode image observation mode by the mode switching unit 37, ultrasound processing according to the aforementioned first embodiment is performed. On the other hand, if the observation mode is set to the flow observation mode by the mode switching unit 37, the first ultrasound processing unit 31 performs ultrasound transmission processing corresponding to the flow observation mode (B mode scanning and flow mode scanning to be described later).

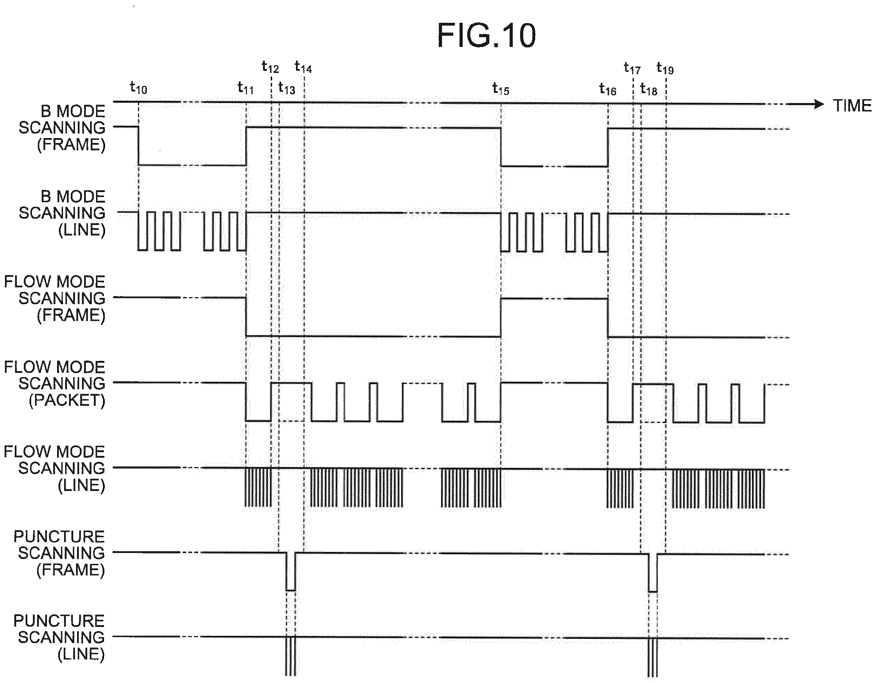

FIG. 10 is a diagram illustrating an ultrasound transmission timing controlled by the ultrasound observation apparatus according to this second embodiment. In addition, in this second embodiment, the description will be given assuming that, in the B mode scanning, line scanning is performed at the time of a fall or a rise of a pulse. In the flow observation mode, when line scanning in the B mode scanning is performed, for acquiring blood flow information on the line, line scanning for the flow mode is performed between frames of the B mode. In the flow mode scanning illustrated in FIG. 10, line scanning for the flow mode is performed eight times in one line. In addition, a plurality of signals obtained by the consecutive line scannings, and forming a pair is referred to as a packet, and the packet is formed for each line. In the ultrasound transmission processing according to this second embodiment, for example, B mode scanning corresponding to one frame ends during a time from a time t₁₀ to a time t₁₁, and flow mode scanning starts from the time t₁₁. If the first line scanning in flow mode scanning of the first frame (scanning corresponding to one packet) is performed in a time from the time t₁₁ to a time t₁₂, puncture scanning starts in a time from a time t₁₃ to a time t₁₄ that corresponds to a period of the next line scanning. After the puncture scanning, the next flow mode scanning of the second line starts. A scanning target line of the flow mode scanning is appropriately set according to blood flow information to be generated. If the last line scanning is performed, a reception signal for the flow mode that corresponds to one frame can be acquired. After the end of the flow mode scanning, the aforementioned processing is repeated, and B mode scanning of the second frame is started in a time from a time t₁₅ to a time t₁₆, and after the end of the B mode scanning, the first line scanning in the flow mode scanning of the second frame (scanning corresponding to one packet) is performed in a time from the time t₁₆ to a time t₁₇. After the first line scanning, puncture scanning is performed in a time from a time t₁₈ to a time t₁₉ that corresponds to a period of the next line scanning. In addition, puncture scanning is preferably performed a plurality of times during the flow mode scanning of one frame. In addition, puncture scanning performed during the B mode scanning is similar to that in the aforementioned first embodiment.

The first signal processing unit 312 obtains a change amount of blood in a blood vessel using eight reception signals (reception signals corresponding to one packet) obtained by line scanning for the flow mode. At this time, blood flow information is generated based on a change amount of amplitude or intensity of each depth. In addition, in line scanning for the flow mode, amplitudes or intensities may be received in all depths of each line, and blood flow information of the entire image may be acquired. Alternatively, blood flow information in a range of a set region of interest (ROI) may be acquired.

The first image generation unit 313 generates B mode image data in which blood flow information is superimposed on B mode image data (hereinafter, referred to as flow mode image data), using B mode reception data generated by the first signal processing unit 312, and blood flow information in each depth. The first image generation unit 313 stores the generated flow mode image data in the first frame memory 314.

In addition, as illustrated in FIG. 10, the second ultrasound processing unit 32 generates puncture needle image data based on the reception signals of puncture scanning that have been acquired at timings between packets in the flow mode scanning. More specifically, first, the second transmission and reception unit 321 transmits a transmission signal (pulse signal) to the ultrasound transducer 21 based on a transmission timing corresponding to a transmission timing of puncture scanning (e.g., time t₁₃, time t₁₈). For example, at the time t₁₃, an ultrasound wave is transmitted from each piezoelectric element, and a plane wave is formed, and the plane wave is transmitted. Ultrasound transmission processing corresponding to one frame of puncture needle image data is thereby completed. By intermittently performing similar ultrasound transmission processing at the time t₁₈ or the like, reception signals for generating puncture image data are acquired during the flow mode scanning, and reception signals for generating puncture needle image data corresponding to a plurality of frames are acquired during ultrasound transmission processing for generating one frame of B mode image data as in the aforementioned first embodiment (e.g., a time from the time t₁₀ to the time t₁₁). After the ultrasound transducer 21 receives an echo signal reflected through such ultrasound transmission processing, puncture needle image data is generated through the second signal processing unit 322 and the second image generation unit 323.

The image synthesis processing unit 33 synthesizes flow mode image data stored in the first frame memory 314, and puncture needle image data stored in the second frame memory 324, to generate synthesized image data. For example, in the synthesized image data IM_{S1} to IM_{S4} illustrated in (b) of FIG. 8, images on which blood flow information is superimposed are obtained.

Synthesized image generated by the aforementioned image synthesis processing is stored in the storage unit 36 and displayed by the display device 4, under the control of the control unit 35. In addition, if the ultrasound observation apparatus 3a receives a new echo signal from the ultrasound endoscope 2, image data of the latest frame of the first frame memory 314 and/or the second frame memory 324 is updated, and image synthesis processing using the updated image data of the latest frame is performed.

According to this second embodiment described above, during ultrasound transmission and reception processing for generating one frame of B mode image data or flow mode image data that is performed by the first ultrasound processing unit 31, the second ultrasound processing unit 32 transmits a plane wave, and generates a plurality of puncture needle image data in which the puncture needle 100 is drawn, and the image synthesis processing unit 33 synthesizes B mode image data or flow mode image data, and puncture needle image data. Synthesized image data including ultrasound images of the puncture needle 100 that have a frame rate shorter than a frame rate of B mode image data is thereby generated. Between frame rates of consecutive B mode image data or between packets of the flow mode, a plurality of ultrasound image data that captures the puncture needle 100 going back and forth with respect to tissue are thereby generated. Even in the case of continuously and swiftly pushing and pulling the puncture needle 100, a movement of the puncture needle 100 can be captured while maintaining resolution of an ultrasound image.

In addition, in the aforementioned second embodiment, the description has been given assuming that puncture scanning is performed at a timing between packets. Nevertheless, in turbo scanning in which a block is formed for a plurality of lines, and flow mode scanning is performed between blocks (one packet is formed across different lines), puncture scanning is performed at a timing between blocks.

### (Third Embodiment)

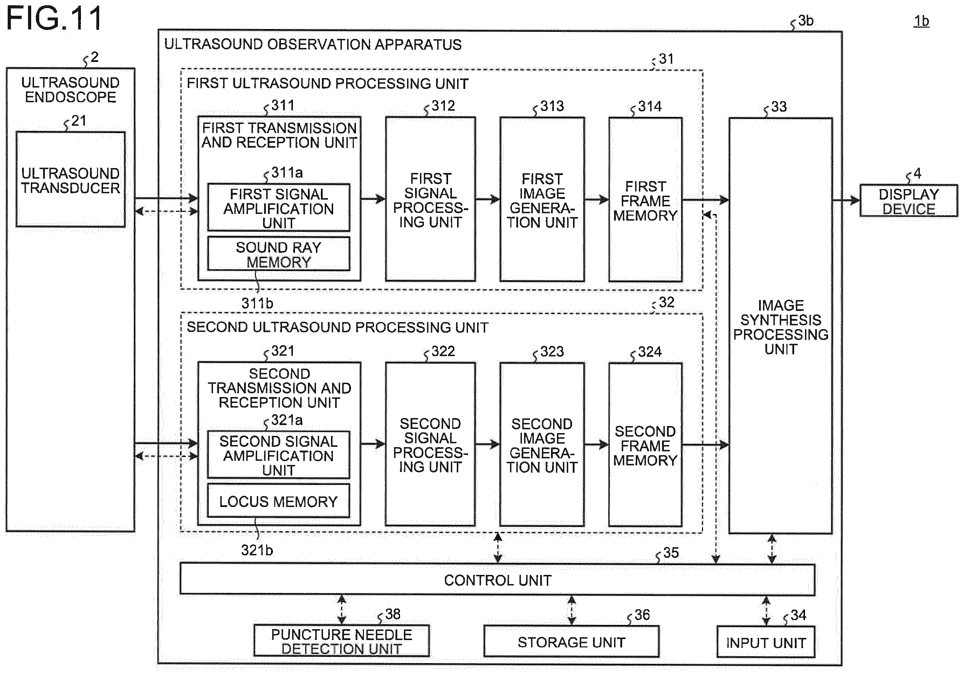

FIG. 11 is a block diagram illustrating a configuration of an ultrasound observation system including an ultrasound observation apparatus according to a third embodiment of the present invention. In this third embodiment, generation of puncture needle image data is controlled by the existence or non-existence of an image of the puncture needle 100 in an ultrasound image.

An ultrasound observation system 1b according to this third embodiment includes an ultrasound endoscope 2 that transmits ultrasound waves to a subject being an observation target, and receives ultrasound waves reflected by the subject, an ultrasound observation apparatus 3b that generates an ultrasound image based on an ultrasound signal acquired by the ultrasound endoscope 2, and a display device 4 that displays the ultrasound image generated by the ultrasound observation apparatus 3b. The ultrasound observation apparatus 3b includes a puncture needle detection unit 38 in addition to the aforementioned configurations of the ultrasound observation apparatus 3.

The puncture needle detection unit 38 performs detection of the puncture needle 100 by referring to the latest B mode image data stored in the first frame memory 314, and determining whether an image of the puncture needle 100 exists in a B mode image corresponding to the B mode image data.

More specifically, if a value of a puncture needle extending position set in a B mode image is different from peripheral values, or has a contrast value larger than a predetermined contrast value, the puncture needle detection unit 38 determines that an image of the puncture needle 100 exists. The puncture needle detection unit 38 outputs existence or non-existence of an image of the puncture needle 100 to the control unit 35 as detection information. The control unit 35 controls the second ultrasound processing unit 32 by determining whether to operate the second ultrasound processing unit 32, according to a detection result of the puncture needle detection unit 38. If an image of the puncture needle 100 is detected by the puncture needle detection unit 38, the control unit 35 causes the second ultrasound processing unit 32 to perform the aforementioned generation processing of puncture needle image data.

According to this third embodiment described above, an effect according to the aforementioned first embodiment can be obtained, and existence of an image of the puncture needle 100 in an ultrasound image is detected, and if the image exists, the second ultrasound processing unit 32 is caused to perform generation of puncture needle image data. Thus, a calculation amount related to the generation of puncture needle image data in a case where an image of the puncture needle 100 does not exist in an ultrasound image can be reduced.

### (Fourth Embodiment)

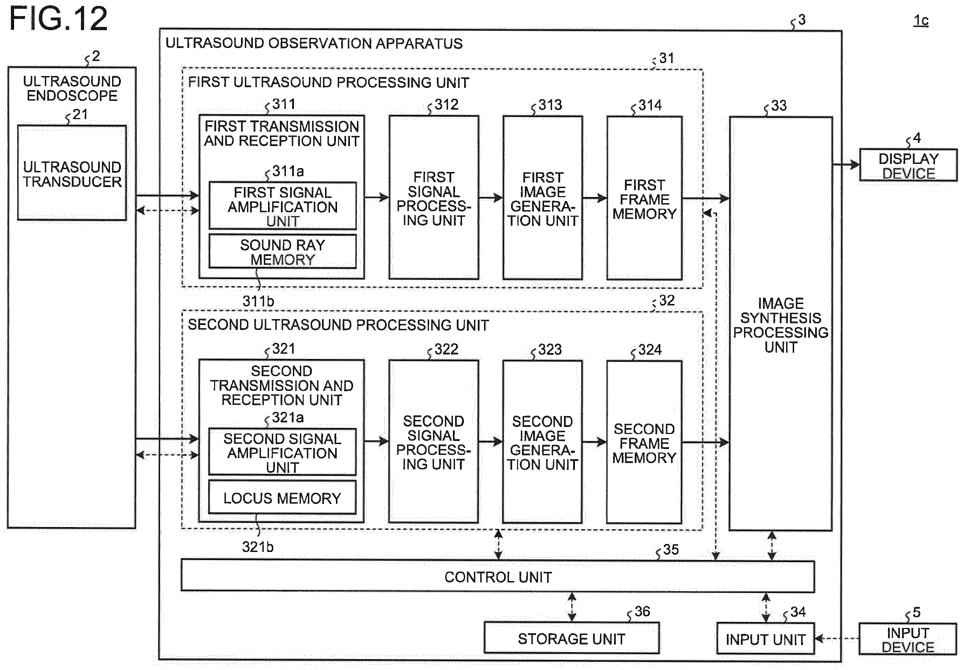

FIG. 12 is a block diagram illustrating a configuration of an ultrasound observation system including an ultrasound observation apparatus according to a fourth embodiment of the present invention. In this fourth embodiment, generation of puncture needle image data and image synthesis processing are performed based on information related to use of the puncture needle 100 that has been received by the input unit 34 via an external input device 5.

An ultrasound observation system 1c according to this fourth embodiment includes an ultrasound endoscope 2 that transmits ultrasound waves to a subject being an observation target, and receives ultrasound waves reflected by the subject, an ultrasound observation apparatus 3 that generates an ultrasound image based on an ultrasound signal acquired by the ultrasound endoscope 2, a display device 4 that displays the ultrasound image generated by the ultrasound observation apparatus 3, and the input device 5 that inputs various types of information to the ultrasound observation apparatus 3.

The input device 5 is implemented by using a user interface such as a keyboard, a mouse, and a touch panel that is electrically connected with the input unit 34, and inputs various types of information to the input unit 34. In this fourth embodiment, the input device 5 can input selection information of enable/disenable of a function of the second ultrasound processing unit 32 to the input unit 34 by pressing a predetermined key of a keyboard, for example.

For example, if the input unit 34 receives, via the input device 5, information indicating that the function of the second ultrasound processing unit 32 is enabled, the control unit 35 causes the second ultrasound processing unit 32 to perform the aforementioned generation processing of puncture needle image data. In contrast to this, if the input unit 34 receives, via the input device 5, information indicating that the function of the second ultrasound processing unit 32 is disenabled, the control unit 35 causes the second ultrasound processing unit 32 not to perform the aforementioned generation processing of puncture needle image data.

According to this fourth embodiment described above, an effect according to the aforementioned first embodiment can be obtained, and availability and unavailability of generation processing of puncture needle image data that is performed by the second ultrasound processing unit 32 is set via the input device 5. Thus, a calculation amount related to the generation of puncture needle image data in a case where generation of puncture needle image data is unnecessary can be reduced.

A mode for carrying other the present invention has been described so far. Nevertheless, the present invention is not to be limited only by the aforementioned embodiments. For example, in an ultrasound observation apparatus, circuits having respective functions may be formed by connecting via buses, or part of the functions may be formed so as to be built in circuit structures of other functions.

In addition, in the present embodiment, the description has been given using an ultrasound endoscope including an optical system such as a light guide, as an ultrasound probe. Nevertheless, the ultrasound probe is not limited to the ultrasound endoscope 2, and an ultrasound probe not including an imaging optical system and an image sensor may be employed. Furthermore, an ultrasound miniature probe not having an optical system and having a thin diameter may be applied as an ultrasound probe. The ultrasound miniature probe is normally inserted into a biliary tract, biliary duct, pancreas duct, trachea, bronchus, urethra, or ureter, and is used when their adjacent organs (pancreas, lung, glandula prostatica, urinary bladder, lymph nodes, etc.) are observed.

In addition, an external ultrasound probe that emits ultrasound waves from a body surface of a subject may be applied as an ultrasound probe. The external ultrasound probe is normally used with being in direct contact with the body surface when abdominal organs (liver, cholecystis, urinary bladder), breast (in particular, glandula mammaria), and glandula thyreoidea are observed.

In addition, the ultrasound transducer may be any of a linear transducer, a radial transducer, and a convex transducer. When the ultrasound transducer is a linear transducer, a scanning region thereof forms a rectangle (oblong, square), and when the ultrasound transducer is a radial transducer or a convex transducer, a scanning region thereof forms a sector shape or a circular shape. In addition, the ultrasound endoscope may mechanically perform scanning of the ultrasound transducer, or may electrically perform scanning by providing, as an ultrasound transducer, a plurality of elements in an array, and electrically switching an element involved in transmission and reception, or delaying transmission and reception of each element.

In this manner, the present invention can include various embodiments without departing from the technical ideas described in the claims.

### Industrial Applicability

As described above, the ultrasound observation apparatus according to the present invention is useful for capturing a movement of a puncture needle while maintaining resolution of an ultrasound image, even in the case of continuously and swiftly pushing and pulling the puncture needle.

### Reference Signs List

1 ULTRASOUND OBSERVATION SYSTEM
2 ULTRASOUND ENDOSCOPE
3 ULTRASOUND OBSERVATION APPARATUS
4 DISPLAY DEVICE
5 INPUT DEVICE
21 ULTRASOUND TRANSDUCER
31 FIRST ULTRASOUND PROCESSING UNIT
32 SECOND ULTRASOUND PROCESSING UNIT
33 IMAGE SYNTHESIS PROCESSING UNIT
34 INPUT UNIT
35 CONTROL UNIT
36 STORAGE UNIT
37 MODE SWITCHING UNIT
38 PUNCTURE NEEDLE DETECTION UNIT
311 FIRST TRANSMISSION AND RECEPTION UNIT
311a FIRST SIGNAL AMPLIFICATION UNIT
311b SOUND RAY MEMORY
312 FIRST SIGNAL PROCESSING UNIT
313 FIRST IMAGE GENERATION UNIT
314 FIRST FRAME MEMORY
321 SECOND TRANSMISSION AND RECEPTION UNIT
321a SECOND SIGNAL AMPLIFICATION UNIT
321b LOCUS MEMORY
322 SECOND SIGNAL PROCESSING UNIT
323 SECOND IMAGE GENERATION UNIT
324 SECOND FRAME MEMORY

## Claims

1. An ultrasound observation apparatus for generating an ultrasound image based on an echo signal obtained by converting an ultrasound echo being an ultrasound wave transmitted onto an observation target and reflected by the observation target, into an electrical signal, the ultrasound observation apparatus comprising:
a first ultrasound processing unit configured to control transmission and reception processing of an ultrasound wave to the observation target, and generate first image data based on an echo signal obtained by the transmission and reception processing;
a second ultrasound processing unit configured to control transmission and reception processing of an ultrasound wave to the observation target by using a scanning method having a frame rate shorter than a frame rate of the first ultrasound processing unit at a timing different from the transmission and reception processing performed by the first ultrasound processing unit, and generate second image data based on an echo signal obtained by the transmission and reception processing; and
an image synthesis processing unit configured to synthesize the first image data generated by the first ultrasound processing unit, and the second image data generated by the second ultrasound processing unit, to generate display image data.

2. The ultrasound observation apparatus according to claim 1, wherein the second ultrasound processing unit generates a plurality of the second image data while the first ultrasound processing unit acquires an echo signal corresponding to one frame of the first image data.

3. The ultrasound observation apparatus according to claim 1,
wherein the first ultrasound processing unit outputs a transmission signal for generating a first transmission echo for performing scanning for each line extending in a predetermined direction, to an ultrasound probe that transmits and receives the ultrasound wave, and outputs the echo signal, and
wherein the second ultrasound processing unit outputs, to the ultrasound probe, a transmission signal for generating a second transmission echo having a range wider than the first transmission echo.

4. The ultrasound observation apparatus according to claim 3, wherein the second transmission echo is a plane wave in which phases are two-dimensionally aligned.

5. The ultrasound observation apparatus according to claim 1, wherein generation processing of the second image data that is performed by the second ultrasound processing unit includes image enhancement processing.

6. The ultrasound observation apparatus according to claim 2, further comprising:
a detection unit configured to detect existence of a drawing target of the second image data in an image corresponding to the first image data, based on the first image data; and
a control unit configured to control whether to operate the second ultrasound processing unit, according to a detection result of the detection unit.

7. The ultrasound observation apparatus according to claim 3, wherein the first and second image data are each generated based on the echo signal obtained by the same ultrasound probe.
